Europäisches Patentamt

European Patent Office

Office européen des brevets

(11) Numéro de publication: **0 197 865**

**A1**

## DEMANDE DE BREVET EUROPEEN

(21) Numéro de dépôt: 86420073.8

(22) Date de dépôt: 13.03.86

(51) Int. Cl.⁴: **G 01 N 21/07**
**G 01 N 33/53**

(30) Priorité: 26.03.85 FR 8504476

(43) Date de publication de la demande:
15.10.86 Bulletin 86/42

(84) Etats contractants désignés:
AT BE CH DE GB IT LI LU NL SE

(71) Demandeur: Guigan, Jean
9, rue Jean Mermoz
F-75008 Paris(FR)

(72) Inventeur: Guigan, Jean
9, rue Jean Mermoz
F-75008 Paris(FR)

(74) Mandataire: Laurent, Michel et al,
20 rue Louis Chirpaz Boîte postale no. 32
F-69131 Ecully Cedex(FR)

(54) Procédé pour réaliser des analyses biologiques utilisant des réactions immunologiques et dispositif de mise en oeuvre.

(57) Procédé pour réaliser des analyses biologiques utilisant des réactions immunologiques et dispositif pour la mise en oeuvre du procédé.

Procédé pour réaliser l'analyse biologique d'un échantillon liquide utilisant un boîtier (1) unitaire en matière plastique, compartimenté de manière à comporter une chambre de stockage (4) pour l'échantillon, une cellule calibrée (6), et des chambres de stockage (30, 40, 50) pour des liquides, tels que conjugué substratet liquide bloquant. Diverses centrifugations permettent de faire passer successivement l'échantillon et lesdits liquides dans une cuve de réaction (11) contenant une bille (17) munie initialement d'antigènes ou d'anticoprs. Des moyens de rinçage de la bille (17) sont également prévus.

FIG.1

EP 0 197 865 A1

0197865

Procédé pour réaliser des analyses biologiques utilisant des réactions immunologiques et dispositif de mise en oeuvre

La présente invention concerne un procédé pour réaliser des analyses biologiques utilisant des réactions immunologiques à support solide, et un dispositif de mise en oeuvre.

On sait que de telles analyses impliquent de nombreuses manipulations de la part de l'opérateur, telles que transferts entre tubes à essais, avec les risques de contamination qui en résultent.

La présente invention a pour but de réaliser un procédé susceptible d'être entièrement automatisé et pouvant être utilisé très simplement par les praticiens eux-mêmes dans leurs cabinets.

La présente invention a pour objet un procédé pour réaliser des analyses biologiques utilisant des réactions immunologiques sur un échantillon liquide, remarquable notamment par le fait qu'il utilise un boîtier compartimenté de façon à présenter :

- une chambre de stockage pour ledit échantillon liquide reliée par un conduit capillaire à une extrémité d'une cellule calibrée dont l'autre extrémité est reliée par un conduit capillaire à une chambre de trop-plein,

- plusieurs chambres de stockage pour des liquides, tels que conjugué, substrat et liquide bloquant, disposées autour d'un déversoir, ledit déversoir étant relié respectivement par des conduits capillaires d'orientations diverses à ladite cellule calibrée et auxdites chambres de stockage de liquides, et communiquant par ailleurs avec une cuve de réaction par l'intermédiaire d'une gouttière interdisant tout refoulement de liquide de cette cuve vers ledit déversoir,

- une cavité d'évacuation de liquide communiquant avec ladite cuve de réaction,

- une bille immobilisée dans ladite cuve de réaction et susceptible d'être couverte d'antigènes ou d'anticorps.

Ledit boîtier est fermé par un couvercle muni d'un réceptacle d'introduction d'échantillon communiquant directement avec ladite chambre de stockage d'échantillon et situé au-dessus d'elle, d'un bouchon amovible entrant dans ledit déversoir pour obturer les extrémités desdits conduits capillaires qui y aboutissent, et enfin d'une

- 2 -

0197865

ouverture obturable communiquant directement avec ladite cuve de réaction pour l'introduction d'un liquide de rinçage.

Des moyens sont prévus pour positionner ledit boîtier sur la périphérie d'un plateau tournant selon plusieurs positions déterminées se déduisant l'une de l'autre par une rotation du boîtier sur lui-même, par rapport au plateau, selon un angle donné.

Selon ce procédé, on a, disposé initialement le conjugué, le substrat et le liquide bloquant, respectivement dans lesdites chambres de stockage de liquides, on introduit ledit échantillon dans ledit réceptacle d'introduction d'échantillon, ce dernier s'écoulant alors par gravité dans ladite chambre de stockage d'échantillon ; on enlève ledit bouchon, on met en place ledit boîtier sur ledit plateau tournant en vue de réaliser successivement plusieurs centrifugations, la position angulaire du boîtier étant à chaque fois choisie, parmi lesdites positions déterminées, en fonction de l'orientation du conduit capillaire concerné par rapport à la direction de la force centrifuge, de manière à effectuer la suite des opérations suivantes :

- passage dudit échantillon de ladite chambre de stockage dans ladite cellule calibrée,

- passage de l'échantillon de ladite cellule calibrée dans ladite cuve de réaction,

- élimination du surplus d'échantillon qui ne s'est pas fixé sur ladite bille vers ladite cavité d'évacuation,

- introduction de liquide de rinçage dans ladite cuve de réaction pour un premier lavage de ladite bille, puis élimination de ce liquide vers ladite cavité d'évacuation,

- introduction dudit conjugué dans ladite cuve de réaction par l'intermédiaire dudit déversoir,

- élimination du surplus dudit conjugué qui ne s'est pas fixé sur ladite bille vers ladite cavité d'évacuation,

- introduction de liquide de rinçage dans ladite cuve de réaction pour un second lavage de ladite bille et élimination de ce liquide vers ladite cavité d'évacuation,

- introduction dudit substrat dans ladite cuve de réaction par l'intermédiaire dudit déversoir,

- élimination du surplus dudit substrat qui ne s'est pas fixé sur ladite bille vers ladite cavité d'évacuation,

- introduction dudit liquide bloquant dans ladite cuve de réaction par l'intermédiaire dudit déversoir.

La réaction au niveau de ladite bille peut être lue alors par tout moyen approprié, par exemple un photomètre, situé au-dessus du couvercle dudit boîtier.

Selon un mode de réalisation particulièrement avantageux, lesdites positions déterminées du boîtier se déduisent les unes des autres par des rotations de l'ordre de 60°, 90° et de 180°, correspondant sensiblement aux angles que font entre eux lesdits conduits capillaires aboutissant dans ledit déversoir.

La présente invention a également pour objet un dispositif pour la mise en oeuvre du procédé précité. Il s'agit d'un boîtier cylindrique plat en matière plastique transparente dont le diamètre est de l'ordre de trois centimètres et qui est compartimenté intérieurement de façon à présenter :

- une chambre de stockage pour ledit échantillon liquide reliée par un conduit capillaire à une extrémité d'une cellule calibrée dont l'autre extrémité est reliée par un conduit capillaire à une chambre de trop-plein,

- plusieurs chambres de stockage pour des liquides disposées autour d'un déversoir, ledit déversoir étant relié respectivement par des conduits capillaires d'orientations diverses à ladite cellule calibrée et auxdites chambres de stockage de liquides, et communiquant par ailleurs avec une cuve de réaction par l'intermédiaire d'une gouttière interdisant tout refoulement de liquide vers ledit déversoir,

- une cavité d'évacuation de liquide communiquant avec ladite cuve de réaction,

- une bille immobilisée dans ladite cuve de réaction.

Ledit boîtier constitue une pièce unitaire venue de moulage. Les conduits capillaires ont des diamètres de l'ordre de deux dizièmes de millimètre.

Le boîtier est fermé par un couvercle en matière plastique transparente muni d'un réceptacle d'introduction d'échantillon communiquant directement avec ladite chambre de stockage d'échantillon et situé au-dessus d'elle. Le couvercle présente en outre une cheminée située au-dessus dudit déversoir et destinée à recevoir un bouchon susceptible d'obturer tous les orifices débouchant dans ledit déversoir. Cette cheminée ainsi que ledit réceptacle constituent avec le couvercle une pièce unitaire en matière plastique, l'ensemble étant venu du moulage.

Une ouverture obturable est également prévue dans le couvercle au-dessus de ladite cuve de réaction.

Selon un mode de réalisation particulièrement avantageux, les conduits capillaires faisant communiquer deux chambres de stockage desdits liquides avec le déversoir sont diamétralement opposés par rapport au déversoir et font respectivement un angle sensiblement égal à 60° et 120° avec le conduit capillaire faisant communiquer la troisième chambre de stockage de liquide et ledit déversoir.

Ledit déversoir comporte avantageusement des nervures internes formant déflecteurs permettant d'éviter qu'un réactif chassé d'une chambre de stockage ne pénètre dans une autre chambre de stockage.

Les chambres de stockage comportent également des nervures internes les compartimentant partiellement et susceptibles de rompre la pente prise par les liquides lors des différentes centrifugations.

Un tel procédé présente l'avantage essentiel de pouvoir être entièrement automatisable et piloté par un système électronique gérant l'ensemble des phases et interprétant les résultats.

D'autres caractéristiques et avantages de la présente invention apparaîtront au cours de la description suivante d'un mode de réalisation donné à titre illustratif, mais nullement limitatif. Dans le dessin annexé :

- la figure 1 est une vue en perspective éclatée d'un dispositif permettant la mise en oeuvre du dispositif selon l'invention,
- la figure 2 est une vue de dessus coupée du boîtier de la figure 1,
- les figures 3 à 20 sont des vues analogues à celle de la figure 2 illustrant les différentes étapes du procédé selon l'invention.

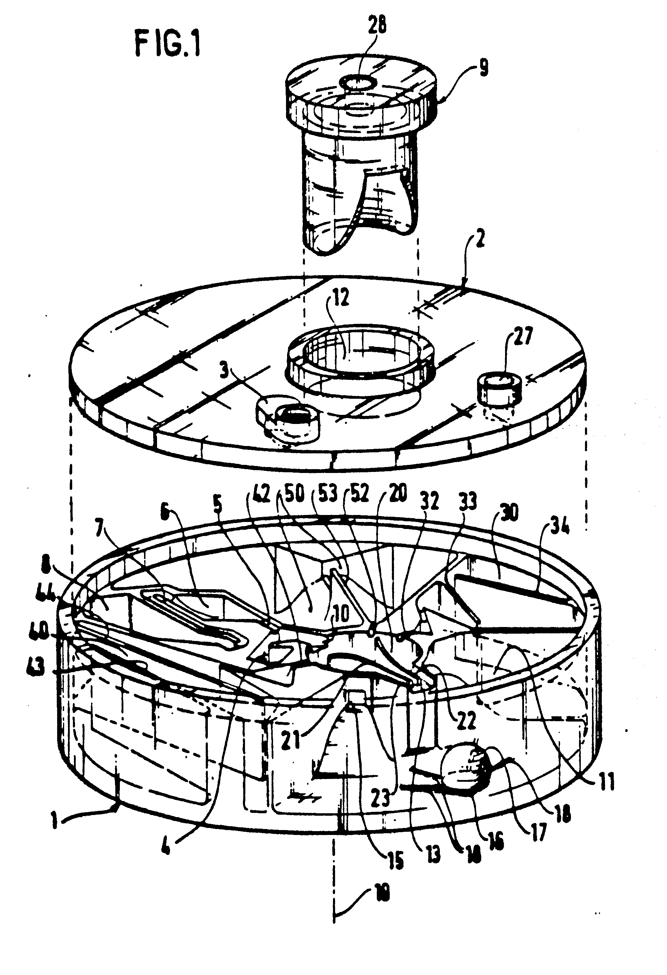

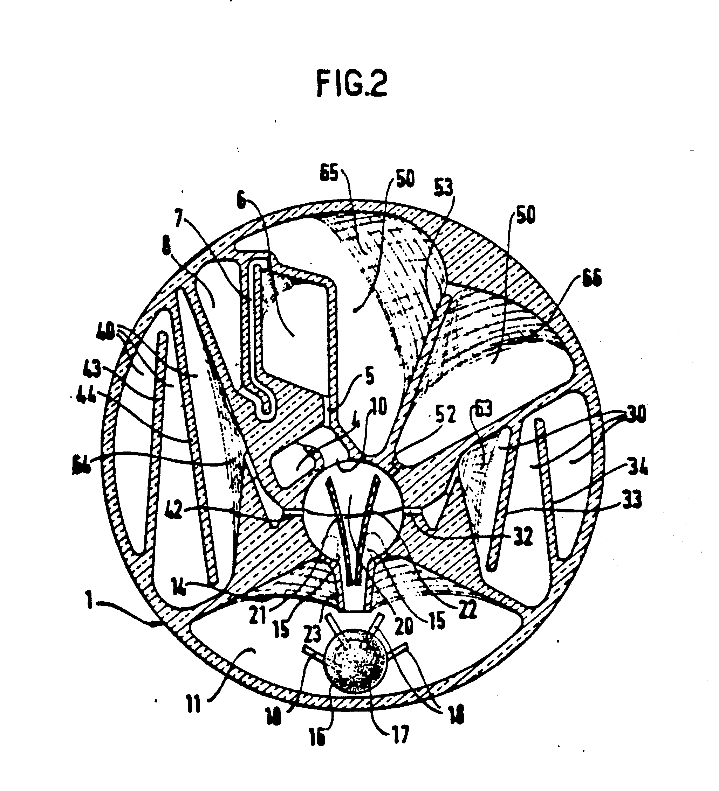

On voit dans les figures 1 et 2 un boîtier 1 de forme générale cylindrique et plate, fermé par un couvercle 2 en matière plastique par exemple par thermosoudage. A titre d'exemple le boîtier a un diamètre de l'ordre de 35 millimètres et une hauteur de l'ordre de 7 millimètres.

La partie supérieure du couvercle 2 porte un réceptacle d'introduction 3 d'un échantillon liquide communiquant directement avec une chambre 4 de stockage d'échantillon intérieure au boîtier.

La chambre de stockage 4 est reliée par un conduit capillaire 5 à une cellule calibrée 6 communiquant par un conduit capillaire 7 avec une chambre de trop-plein 8, et par un orifice 10 avec un déversoir 20. Ce dernier communique avec une cuve de réaction 11 par une gouttière 23 définissant avec le couvercle 2 un orifice 13. Son niveau est tel qu'il permet un écoulement de liquide du déversoir 20 vers la cuve de réaction 11, et qu'il empêche tout passage inverse de liquide, même en cas de centrifugation. Le déversoir est muni de nervures déflectrices 21 et 22 qui guident les liquides sortant des chambres vers la cuve de réaction 11.

La cuve de réaction 11 présente, du côté du déversoir 20, une paroi inclinée 14 munie à sa partie supérieure d'orifices 15 communiquant avec une cavité interne d'évacuation de liquide. Le fond de la cuve de réaction 11 comporte un logement 16 en forme de calotte sphérique pour une bille 17. On a référencé 18 quelques rainures aboutissant dans le logement 16. A titre d'exemple, la bille a un diamètre de 6 mm.

Le boîtier comporte en outre trois chambres de stockage pour des liquides :

- la chambre de stockage 30 pour un conjugué 31, communiquant avec le déversoir 20 par un conduit capillaire 32, et partiellement fractionné par des parois internes 33 et 34,

- la chambre de stockage 40 pour un substrat 41, communiquant avec le déversoir 20 par un conduit capillaire 42, et partiellement fractionné par des parois internes 43 et 44,

- la chambre de stockage 50 pour un liquide bloquant 51 communiquant avec le déversoir 20 par un conduit capillaire 52, et fractionné partiellement par une paroi interne 53.

On a référencé 63, 64, 65 et 66 les pentes de certaines parois appartenant respectivement aux chambres 30, 40 et 50.

A titre d'exemple, ces chambres ont des volumes respectifs de 200, 300, et 300 microlitres. Les conduits capillaires 5, 32, 42, 52 présentent diverses orientations par rapport au déversoir 20. Les conduits 32 et 42 sont sensiblement diamètralement opposés, le conduit 5 est sensiblement orthogonal aux deux précédents, tandis que le conduit 52 forme un angle sensiblement égal à 45 degrés avec le conduit 32.

Le couvercle 2 porte au-dessus du déversoir 20 une cheminée 12 susceptible de recevoir un bouchon 9. Pendant toute la durée de conservation du boîtier muni de ses liquides, le bouchon 9 a pour fonction d'obturer tous les orifices débouchant dans le déversoir 20 (conduits capillaires 32, 42, 52 et orifice 13 notamment). On a référencé 28 un orifice d'aération prévu dans le bouchon 9.

Enfin le couvercle 2 est muni d'une ouverture obturable 27, située au-dessus de la cuve de réaction, et par où il est possible d'introduire directement de l'eau ou tout autre liquide de rinçage.

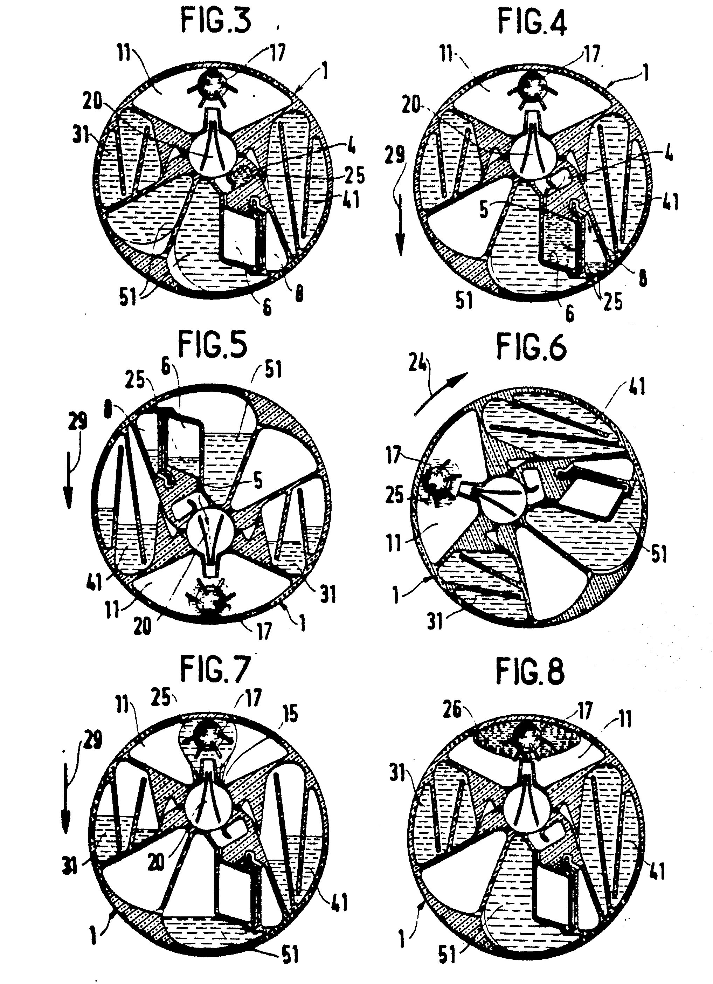

Les figures 3 à 20 montrent en vue de dessus le boîtier 1 selon l'invention. Il est initialement rempli des trois liquides, conjugué 31, substrat 41 et liquide bloquant 51, et la bille 17 est recouverte d'antigènes ou d'anticorps selon la réaction à étudier. Fermé hermétiquement par son couvercle 2 et son bouchon 9, le boîtier 1 peut être stocké ainsi, prêt à l'emploi.

Au moment de l'utilisation on introduit une certaine quantité de sérum 25 dans le réceptacle d'introduction 3 ; le sérum 25 s'écoule par gravité dans la chambre de stockage d'échantillon 4.

On dispose alors le boîtier sur la couronne d'un plateau tournant pour y subir plusieurs centrifugations. Les figures 5, 7, 9, 15, 18 illustrent ces phases de centrifugation, la direction de la force centrifuge étant toujours représentée par les flèches 29. Entre deux centrifugations le boîtier est susceptible de subir des rotations autour de son axe 19, indiquées par les flèches 24.

La figure 3 montre la position initiale du boîtier 1. Au cours d'une première centrifugation, le sérum 25 passe (voir figure 4) de la chambre de stockage d'échantillon 4, par le conduit capillaire 5 vers la cellule calibrée 6, le conduit capillaire 7 et la chambre de trop-plein 8. La cellule calibrée 6 contient par exemple 200 microlitres de sérum.

Après avoir subi une rotation de 180° autour de son axe, et une seconde centrifugation (figure 5) le volume de sérum 25 contenu dans la cellule calibrée 6 repasse par le conduit capillaire 5 et le déversoir 20 pour aboutir sur la bille 17.

Après une nouvelle rotation de 180° (figures 6 et 7), une troisième centrifugation (figure 7) fait passer le surplus de sérum 25 par les orifices 15 dans la cuve d'évacuation de liquide.

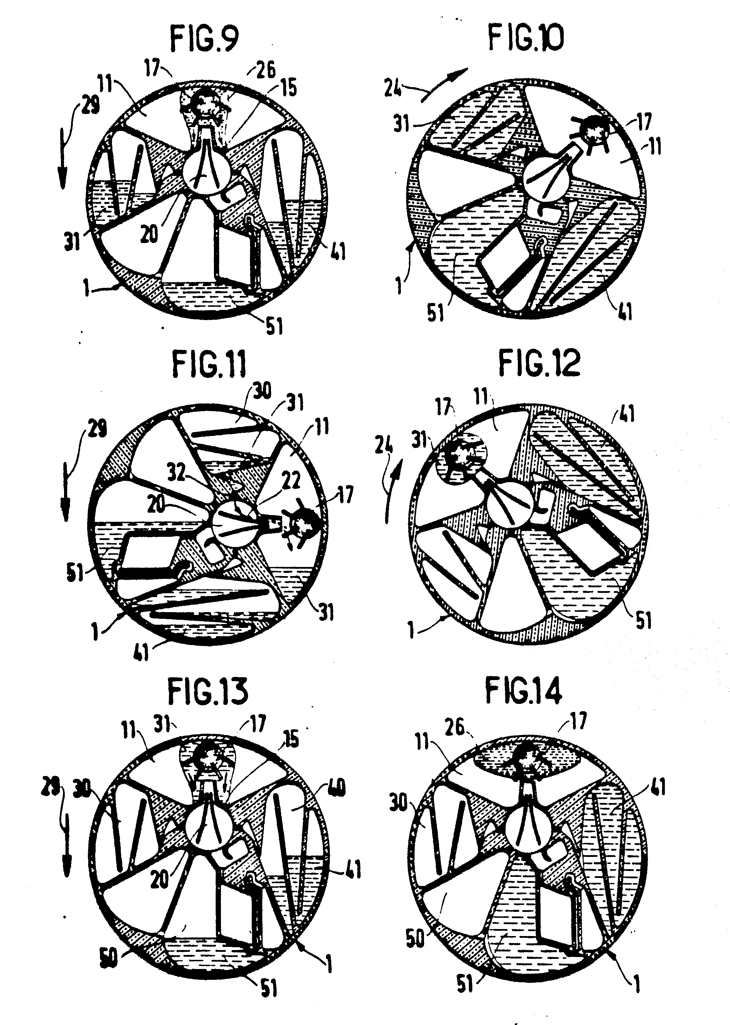

Dans la phase de la figure 8 on introduit par l'ouverture 27 du couvercle 2 et l'intermédiaire d'une pipette calibrée une quantité d'eau 26 directement dans la cuve de réaction 11. Comme le montre la figure 9, une quatrième centrifugation permet l'élimination du surplus d'eau 26 dans la cuve d'évacuation de liquide par les orifices 15.

Les figures 10 et 11 montrent que l'on fait tourner le boîtier 1 de 90 degrés. Dans cette position le conduit capillaire 32 se trouve parallèle à la direction 29 de la force centrifuge. Le liquide conjugué 31 de la chambre 30 passe au cours d'une cinquième centrifugation par le conduit 32 dans la cuve de réaction 11. Une nouvelle rotation de 90 degrés (figures 12 et 13) permet à la bille de baigner dans le conjugué 31. La sixième centrifugation illustrée par la figure 13, a pour effet d'éliminer le trop-plein de conjugué 31 par les orifices 15 vers la cuve d'évacuation.

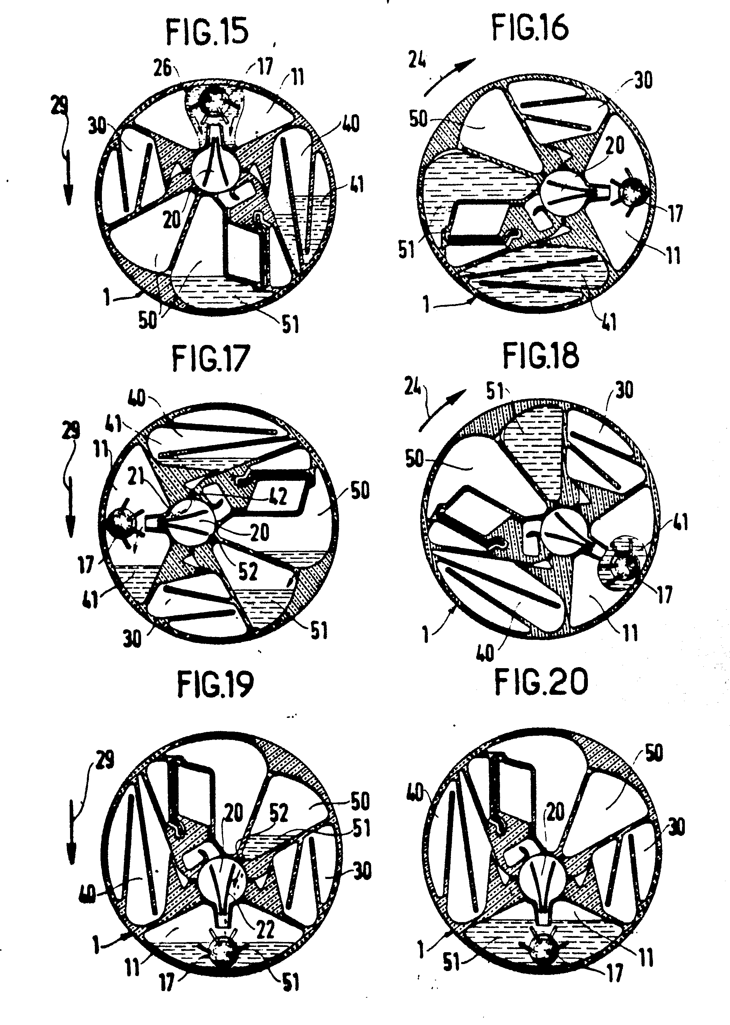

Dans la phase illustrée par la figure 14, on introduit par l'ouverture 27 du couvercle une nouvelle quantité d'eau 26 dans la cuve de réaction 11. La septième centrifugation montrée dans la figure 15 élimine après rinçage le surplus d'eau par les orifices 15 vers la cuve d'évacuation.

On effectue ensuite une rotation de 270 degrés (figures 16 et 17), de manière à ce que le conduit capillaire 42 se trouve parallèle à la direction de la force centrifuge 29. Une huitième centrifugation a pour effet de faire passer le substrat 41 de la cuve 40 par le conduit capillaire 42, dans le déversoir 20 et la cuve de réaction 11. Simultanément, le liquide bloquant passe dans le compartiment de la chambre 50 qui comporte le conduit capillaire 52.

On réalise une nouvelle rotation d'au moins 270 degrés (voir figures 18 et 19). La direction du conduit capillaire 52 est suffisamment proche de celle de la force centrifuge 29 pour que le liquide bloquant 51 passe par ce conduit et le déversoir 20 vers la cuve de réaction 11.

L'état final est illustré par la figure 20 ; toutes les chambres sont vides et la réaction qui a lieu au niveau de la bille peut être observée à travers le couvercle 2, par exemple par un photomètre.

Toutes les phases du procédé qui viennent d'être décrites peuvent être automatisées aisément.

Bien entendu, l'invention n'est pas limitée au mode de réalisation qui vient d'être décrit. Ainsi, il est possible de ne pas utiliser la bille et d'enduire la cavité où elle est logée d'anticorps ou d'antigènes. On peut aussi utiliser plusieurs billes immobilisées dans la cuve de réaction. Le liquide bloquant peut être un simple diluant. On pourra, sans sortir du cadre de l'invention, remplacer tout moyen par un moyen équivalent.

0197865

## REVENDICATIONS

1/ Procédé pour réaliser des analyses biologiques utilisant des réactions immunologiques sur un échantillon liquide, caractérisé par le fait :

- qu'il met en oeuvre un boitier (1) fermé par un couvercle (2), ledit boitier (1) étant compartimenté de façon à présenter :

. une chambre de stockage (4) pour ledit échantillon liquide reliée par un conduit capillaire (5) à une extrémité d'une cellule calibrée (6) dont l'autre extrémité est reliée par un conduit capillaire (7) à une chambre de trop-plein (8),

. plusieurs chambres de stockage (30,40,50) pour des liquides, tels que conjugué (31), substrat (41) et liquide bloquant (51), disposées autour d'un déversoir (20), ledit déversoir étant relié respectivement par des conduits capillaires (32,42,52) d'orientations diverses à ladite cellule calibrée (6) et auxdites chambres de stockage de liquides (30, 40,50), et communiquant par ailleurs avec une cuve de réaction (11) par l'intermédiaire d'une gouttière (23) interdisant tout refoulement de liquide de cette cuve vers ledit déversoir (20),

. une cavité d'évacuation de liquide communiquant avec ladite cuve de réaction (11),

. une bille (17) immobilisée dans ladite cuve de réaction (11) et susceptible d'être couverte d'antigènes ou d'anticorps,

ledit couvercle étant muni :

. d'un réceptacle d'introduction, d'échantillon communiquant directement avec ladite chambre de stockage d'échantillon et situé

- 10 -

au-dessus d'elle,

. d'un bouchon amovible (9) entrant dans ledit déversoir (20) pour obturer les extrémités desdits conduits capillaires (32,42,52) qui y aboutissent,

. d'une ouverture obturable (27) communiquant directement avec ladite cuve de réaction (11) pour l'introduction d'un liquide de rinçage,

- que des moyens sont prévus pour positionner ledit boitier sur la périphérie d'un plateau tournant selon plusieurs positions déterminées se déduisant l'une de l'autre par une rotation du boitier sur lui-même, par rapport au plateau, selon un angle donné,

- que l'on a disposé initialement ledit conjugué (31), ledit substrat (41) et ledit liquide bloquant (51) respectivement dans lesdites chambres de stockage (30, 40,50),

- que l'on introduit ledit échantillon dans ledit réceptacle d'introduction d'échantillon, ce dernier s'écoulant alors par gravité dans ladite chambre de stockage (4) d'échantillon,

- que l'on enlève ledit bouchon (9),

- que l'on met en place ledit boitier (1) sur ledit plateau tournant en vue de réaliser successivement plusieurs centrifugations, la position angulaire du boitier (1) étant à chaque fois choisie, parmi lesdites positions déterminées, en fonction de l'orientation du conduit capillaire concerné par rapport à la direction de la force centrifuge, de manière à effectuer la suite des opérations suivantes :

. passage dudit échantillon de ladite chambre de stockage (4) dans ladite cellule calibrée (6),

. passage dudit échantillon de ladite cellule (6) calibrée dans ladite cuve de réaction (11),

. élimination du surplus d'échantillon qui ne s'est pas fixé sur ladite bille (17) vers ladite cavité d'évacuation,

. introduction dudit liquide de rinçage dans ladite cuve de réaction (11) pour un premier
lavage de la bille (17), puis élimination de
ce liquide vers ladite cavité d'évacuation,

. introduction dudit conjugué (31) dans ladite
cuve de réaction (11) par l'intermédiaire dudit déversoir (20),

. élimination du surplus dudit conjugué (31) qui
ne s'est pas fixé sur ladite bille (17) vers
ladite cavité d'évacuation,

. introduction dudit liquide de rinçage dans
ladite cuve de réaction (11) pour un second
lavage de ladite bille (17) et élimination dudit liquide vers ladite cavité d'évacuation,

. introduction dudit substrat (41) dans ladite
cuve de réaction (11) par l'intermédiaire dudit déversoir (20),

. élimination du surplus dudit substrat (41) qui
ne s'est pas fixé sur ladite bille (17) vers
ladite cavité d'évacuation,

. introduction dudit liquide bloquant (51) dans
ladite cuve de réaction (11) par l'intermédiaire dudit déversoir (20),

- et que la réaction au niveau de ladite bille est
lue alors par un moyen optique d'analyse.

2/ Procédé selon la revendication 1, caractérisé par
le fait que lesdites positions déterminées du boitier (1)
se déduisent les unes des autres par des rotations de l'ordre de 60°, 90°, 180° et de 270° correspondant sensiblement aux angles que font entre eux lesdits conduits capillaires (32,42,52) aboutissant dans ledit déversoir (20).

3/ Dispositif pour la mise en oeuvre du procédé selon
l'une des revendications 1 et 2, caractérisé par le fait qu'il comprend un boitier (1) cylindrique plat en matière plastique transparente fermé par un couvercle (2) en matière plastique transparente, ledit boitier (1) moulé étant d'une seule pièce compartimentée intérieurement de façon à présenter :

- une chambre de stockage (4) pour ledit échantillon liquide reliée par un conduit capillaire (5) à une extrémité d'une cellule calibrée (6) dont l'autre extrémité est reliée par un conduit capillaire(7) à une chambre de trop-plein (8),

- plusieurs chambres de stockage (30,40,50) pour des liquides disposées autour d'un déversoir (20), ledit déversoir (20) étant relié respectivement par des conduits capillaires (32,42,52) d'orientations diverses à ladite cellule calibrée (6) et auxdites chambres de stockage (30,40,50) de liquide, et communiquant par ailleurs avec une cuve de réaction (11) par l'intermédiaire d'une gouttière (23) interdisant tout refoulement de liquide vers ledit déversoir (20),

- une cavité d'évacuation de liquide communiquant par des orifices avec ladite cuve de réaction (11),

- une bille (17) immobilisée dans ladite cuve de réaction (11),
ledit couvercle (2) étant muni :

- d'un réceptacle d'introduction d'échantillon communiquant directement avec ladite chambre de stockage (4) d'échantillon et situé au-dessus d'elle,

- d'une ouverture obturable (27) située au-dessus de ladite cuve de réaction (11) pour l'introduction dudit liquide de rinçage,

- d'une cheminée (12) située au-dessus dudit déversoir (20) et destinée à recevoir un bouchon (9) susceptible d'obturer tous les orifices débouchant dans ledit déversoir (20), ladite cheminée (12) ainsi que ledit réceptacle constituant avec ledit couvercle (2) une pièce unitaire en matière plastique, l'ensemble étant venu du moulage.

4/ Dispositif selon la revendication 3, caractérisé par le fait que les conduits capillaires (32,42) faisant communiquer deux desdites chambres (30,40) de stockage de liquide avec ledit déversoir (20) sont diamètralement opposés par rapport au déversoir (20) et font respectivement un angle sensiblement égal à 60° et 120° avec le conduit capillaire (52) faisant communiquer ladite troisième chambre (50) de stockage de liquide et ledit déversoir (20).

5/ Dispositif selon l'une des revendications 3 et 4, caractérisé par le fait que ledit déversoir (20) comporte des nervures internes (21,22) formant déflecteurs permettant d'éviter qu'un liquide chassé d'une desdites chambres de stockage (30,40,50) de liquide ne pénètre dans une autre chambre de stockage de liquide.

6/ Dispositif selon l'une des revendications 3 à 5, caractérisé par le fait que lesdites chambres (30,40,50) de stockage de liquide comportent des nervures internes (33,34,43,44,53) les compartimentant partiellement et susceptibles de rompre la pente prise par le liquide lors des différentes centrifugations.

7/ Dispositif selon l'une des revendications 3 à 6, caractérisé par le fait que ledit boitier (1) présente un diamètre de l'ordre de 30 mm, une hauteur de l'ordre de 7 mm, et que ladite bille (17) présente un diamètre de l'ordre de 6 mm .

FIG.1

0197865

- 1/5

FIG.2

## FIG.3

## FIG.4

## FIG.5

## FIG.6

## FIG.7

## FIG.8

FIG.9
FIG.10
FIG.11
FIG.12
FIG.13
FIG.14

0197865

- 4/5

0197865

FIG.15

FIG.16

FIG.17

FIG.18

FIG.19

FIG.20

Office européen
des brevets

**RAPPORT DE RECHERCHE EUROPEENNE**

0197865

Numéro de la demande

EP 86 42 0073

## DOCUMENTS CONSIDERES COMME PERTINENTS

| Catégorie | Citation du document avec indication. en cas de besoin, des parties pertinentes | Revendication concernée | CLASSEMENT DE LA DEMANDE (Int Cl 4) |
|---|---|---|---|
| A | EP-A-0 062 907 (J. GUIGAN) * Abrégé; figures 1-4 * | 1 | G 01 N 21/07 G 01 N 33/53 |
| | --- | | |
| A | FR-A-2 524 874 (J. GUIGAN) * Page 8, lignes 2-13; figures 1A,1B * | 1 | |
| | --- | | |
| A | EP-A-0 106 536 (ORTHO DIAGNOSTIC SYSTEMS) | | |
| | ----- | | |

DOMAINES TECHNIQUES RECHERCHES (Int. Cl 4)

G 01 N 21/00
G 01 N 33/00

Le présent rapport de recherche a été établi pour toutes les revendications

| Lieu de la recherche | Date d'achèvement de la recherche | Examinateur |
|---|---|---|
| LA HAYE | 26-06-1986 | ANTHONY R.G. |